Europäisches Patentamt

European Patent Office

Office européen des brevets

(11) Numéro de publication: **0 506 563 B1**

(12) **FASCICULE DE BREVET EUROPEEN**

(45) Date de publication de fascicule du brevet: **04.01.95**

(51) Int. Cl.⁶: **A61K 31/725**, A61K 33/06, A61K 9/10, A61K 9/22, A61K 47/36

(21) Numéro de dépôt: **92400830.3**

(22) Date de dépôt: **26.03.92**

(54) **Suspension à base d'acide alginique.**

(30) Priorité: **29.03.91 FR 9103894**

(43) Date de publication de la demande:
**30.09.92 Bulletin 92/40**

(45) Mention de la délivrance du brevet:
**04.01.95 Bulletin 95/01**

(84) Etats contractants désignés:
**AT BE CH DE DK ES FR GB GR IT LI LU MC NL PT SE**

(56) Documents cités:
**WO-A-87/05217**
**FR-A- 2 636 532**

(73) Titulaire: **PIERRE FABRE MEDICAMENT**
**45, Place Abel Gance**
**F-92100 Boulogne (FR)**

(72) Inventeur: **Davin, Hervé**
**Montlédier**
**F-81660 Pont de l'Arn (FR)**
Inventeur: **Dubois, Jacques**
**10 Bd Gambetta**
**F-11100 Narbonne (FR)**

(74) Mandataire: **Ahner, Francis et al**
**CABINET REGIMBEAU**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

EP 0 506 563 B1

**Description**

La présente invention concerne une composition pharmaceutique, sous forme de suspension, destinée notamment au traitement des affections gastro-oesophagiennes, plus particulièrement des troubles inflammatoires et irritatifs liés au reflux gastrique et plus spécialement de la hernie hiatale.

Le traitement des affections liées au reflux gastro-oesophagien recourt classiquement à l'administration de substances anti-acides : carbonates ou bicarbonates alcalins ou alcalino-terreux, ou hydroxyde d'aluminium.

L'intérêt de cette thérapeutique est controversé en raison de l'absorption de quantités importantes de sels minéraux :sodium, calcium, magnésium ou aluminium et des risques biologiques et toxicologiques qui en découlent. Elle n'apporte en outre qu'un répit limité dans le temps. Elle induit fréquemment à un "effet-rebond", redoutable dans le type d'affection visée.

Plus récemment, diverses préparations ont été proposées pour supprimer le reflux gastro-oesophagien : elles combinent l'alginate de sodium, le bicarbonate de sodium et le carbonate de calcium en proportions adéquates.

Il est préférable de disposer de compositions sous forme de suspensions buvables, prêtes à l'emploi, qui dispenseront le patient de toute procédure de préparation extemporanée, indispensable pour des formes comprimés ou poudre pour suspensions, et qui peut apparaître fastidieuse ou susceptible d'être mal ou incomplètement réalisée. Ces compositions sous forme de suspensions buvables réagissent avec l'acide chlorhydrique du contenu gastrique pour former une mousse par inclusion du gaz carbonique dans le gel d'acide alginique. Cette mousse surnage au-dessus du contenu gastrique ; elle présente un pH proche de la neutralité. Lors d'un reflux gastro-oesophagien, elle recouvre la muqueuse digestive, la protégeant du contact direct avec le contenu gastrique acide.

Toutefois, de telles préparations pour être efficaces, doivent présenter une viscosité définie et stable. Cette caractéristique implique l'utilisation d'agents viscosants et d'agents de suspension permettant la formation de la mousse et le maintien en dispersion du carbonate de calcium. Ce problème technique a été résolu par l'addition d'ingrédients supplémentaires : il s'agit en particulier, d'un polymère acrylique réticulé avec allyl-saccharose (carbopol) et de pectine ou d'amidon de pomme de terre pré-gélatinisé (P rejet 97) ou de glycalate d'amidon de sodium (primojel).

Ce procédé, outre qu'il implique l'emploi de composants ne concernant pas l'effet thérapeutique visé, présente des inconvénients et des limites dues à l'instabilité de certains viscosants en milieu dont le pH est alcalin, en raison de la présence de bicarbonate de sodium, ou aux risques d'interactions entre les agents de suspension de caractère polaire avec diverses substances actives qui peuvent être ajoutées à la suspension.

C'est pourquoi la présente invention a pour objet une composition pharmaceutique sous forme de suspension, caractérisée en ce qu'elle contient de l'acide alginique à des concentrations comprises entre 2 et 7 % (p/v), du bicarbonate de sodium à des concentrations comprises entre 1,5 et 7 % (p/v) et au moins un agent viscosant choisi dans le groupe comprenant la gomme xanthane, l'alginate de magnésium, et le glycérol.

Cette composition, sous forme liquide ou pâteuse est destinée plus particulièrement au traitement des affections gastro-oesophagiennes liées au reflux gastro-oesophagien.

De préférence, on utilisera de l'acide alginique de viscosité comprise entre 5 et 30 centipoises ; cette viscosité est déterminée avec un viscosimètre de Brookfield, en utilisant une vitesse de rotation de 20 t/min. à 20°C, dans une solution aqueuse à 1 %.

Les qualités particulères des produits obtenus grâce a l'association d'agents viscosants avec l'acide alginique et le bicarbonate de sodium, peuvent être mises en évidence aussi bien in vitro qu'in vivo.

In vitro, la réalisation du test de mousse, désigné couramment par le terme "test du radeau", permet de reconstituer artificiellement le phénomène qui se produit naturellement au niveau de l'estomac. Sa mise en oeuvre consiste à verser 25 ml de suspension dans un bécher contenant 150 ml de solution d'acide chlorhydrique 0,1 N. Après production de l'effervescence, on observe la formation d'une mousse surnageante, dont la qualité est variable selon les formulations testées. Trois paramètres sont évalués :
- la durée de persistance de la mousse : en l'absence de viscosant, la mousse tend à se déliter progressivement puis à disparaître ; ce phénomène se produit dans un délai de 2 heures environ, en l'absence de toute agitation.

Les suspensions formulées avec au moins un des agents viscosants cités ci-dessus comme adjuvant de viscosité, permettent d'obtenir une mousse qui persiste jusqu'à 10 heures dans les conditions expérimentales.

- rigidité de la mousse : qui traduit la fermeté et la stabilité de l'émulsion formée par le gaz carbonique emprisonné dans le gel ; elle peut être évaluée au toucher.

L'action d'un des agents viscosants cités ci-dessus est un facteur essentiel pour l'obtention d'une mousse de structure fine, homogène, régulière et dure, par comparaison avec les formules qui ne les contiennent pas.

- l'épaisseur du radeau formé 30 minutes après le commencement de la réaction varie aussi en fonction des formules. En l'absence d'agent viscosant, l'épaisseur du radeau est d'environ 2 à 2,5 cm. Dans les mêmes conditions opératoires, en présence de l'agent viscosant, cette épaisseur atteint 4 à 4,5 cm.

En particulier, la présente invention a pour objet une composition pharmaceutique sous forme de suspension, caractérisée, en ce qu'elle comporte à titre de principe actif de l'acide alginique, de l'hydroxyde d'aluminium colloïdal, du carbonate de magnésium, et de la silice hydratée, et en ce qu'elle comporte en outre une faible proportion d'au moins un agent viscosant choisi dans le groupe comprenant la gomme xanthane, l'alginate de magnésium, et le glycérol.

Dans un mode de réalisation préférée, la composition pharmaceutique selon la présente invention contient à titre d'agent viscosant, de l'alginate de magnésium, dans une proportion comprise entre 0,1 et 2 %, et de manière encore préférée dans une proportion au plus égale à 0,25 % (p/v).

L'alginate de magnésium a une viscosité comprise entre 400 et 450 centipoises, déterminée au viscosimètre de Brookfield, à 60 tours par minutes, à 20°C, sur une solution aqueuse à 1%. L'alginate de magnésium, à faible concentration, stabilise la suspension et contribue à l'obtention d'une viscosité assurant le maintient en suspension des différentes poudres insolubles durant la répartition et la prise thérapeutique.

Dans cette composition, l'acide alginique et le bicarbonate de sodium réagissent avec l'acide chlorhydrique du contenu gastrique et grâce à la présence d'alginate de magnésium forment la mousse protectrice de la muqueuse gastro-oesophagienne. L'alginate de magnésium permet la formation d'une mousse efficace, stable et rigide. Son association avec l'acide alginique et le bicarbonate de sodium permet d'obtenir une mousse dont les qualités thérapeutiques et la stabilité sont largement supérieures à celles d'une composition incluant les deux seuls autres constituants.

L'alginate de magnésium permet en outre d'ajuster la viscosité du milieu de sorte que la mousse produite dans la cavité gastrique possède des propriétés adéquates ; pour cela, sa concentration, toujours faible, et sa viscosité, sont définies en fonction de la concentration et de la viscosité de l'acide alginique combiné dans la préparation.

En outre, il permet la mise en suspension stable, d'autres agents actifs pouvant être ajoutés à la composition de base. Ainsi, la suspension buvable peut-elle être utilisée seule pour ses propriétés d'ordre physique ou mécanique, mais elle se prête également à l'inclusion de divers agents actifs supplémentaires, jouant en cela le rôle de véhicule stable.

Parmi les agents envisagés, on peut citer :
. le carbonate de calcium ou de magnésium,
. l'hydroxyde d'aluminium,
. la silice colloïdale,
. des substances anesthésiques locales comme benzocaine, xylocaïne, etc,
. des substances anti-inflammatoires : par exemple acide acéxamique ou l'un de ses dérivés, l'acide 18-$\beta$ hydroxy-glycyrrhétinique, ou l'un de ses dérivés, le sucralfate,
. des substances adsorbantes ou filmogènes telles que des montmorillantes ou des silico-aluminates complexes, ou des agents tels que la polyvinyl-pyrrolidone.

La viscosité des préparations formées avec l'acide alginique et donc la stabilité du "radeau", dépend entre autre de la longueur des chaînes d'acide uronique qu'il contient.

Selon un aspect préféré de l'invention, la composition pharmaceutique telle qu'elle a été définie, est caractérisée en ce qu'elle contient du bicarbonate de sodium dans une proportion de 2,5 % (p/v).

Selon un des aspects encore préférés de l'invention, la composition pharmaceutique est caractérisée en ce qu'une unité de dosage de 10 ml de suspension contient :

| - acide alginique | 0,40 g |
| - hydroxyde d'aluminium colloïdal | 0,06 g |
| - carbonate de magnésium | 0,08 g et |
| - silice précipitée | 0,26 g |

La composition pharmaceutique selon l'invention peut contenir en outre des agents édulcorants, aromatisants, et des conservateurs pharmaceutiquement acceptables.

Selon un de ses aspects particulièrement préféré, l'invention a pour objet une composition pharmaceutique caractérisée en ce qu'elle répond à la formule suivante :

| | |
|---|---|
| - acide alginique | 4,000 g |
| - hydroxyde d'aluminium colloïdal | 0,600 g |
| - silice hydratée | 2,600 g |
| - carbonate de magnésium | 0,800 g |
| - alginate de magnésium | 0,250 g |
| - carbonate de calcium | 1,200 g |
| - glycérol | 12,000 g |
| - saccharine sodique | 0,050 g |
| - arôme caramel | 0,010 g |
| - arôme menthe poivrée | 0,0015 g |
| - acide sorbique | 0,190 g |
| - parahydroxybenzoate de méthyle sodé | 0,090 g |
| - parahydroxybenzoate de propyle sodé | 0,045 g |
| - bicarbonate de sodium | 2,500 g |
| - eau purifiée qsp | 100 ml |

et en ce qu'elle contient en outre environ 1 g d'hydroxyde de sodium, de manière à amener le pH à une valeur d'environ 8,2.

Les compositions selon l'invention donnent d'excellents résultats au test in vitro, avec le modèle de l'estomac artificiel, et ces résultats sont confirmés in vivo par des observations cliniques.

Dans tous les cas exploratoires étudiés en vue d'orienter le choix entre les diverses variantes de formulation, il est apparu que les suspensions contenant comme excipient un agent viscosant apportaient un soulagement des douleurs gastriques sensiblement plus prolongées que les formules n'en contenant pas.

Les exemples suivants sont destinés à illustrer l'invention, sans aucunement en limiter la portée.

**EXEMPLE 1**

On prépare une suspension dans laquelle les principes actifs respectent les proportions suivantes :

| | | |
|---|---|---|
| - acide alginique | 0,400 g | |
| - hydroxyde d'aluminium colloïdal | 0,060 g | 1 dose |
| - carbonate de magnésium | 0,080 g | = |
| - silice précipitée | 0,260 g | 10 ml |

En milieu gastrique acide, l'action de ces composants se combine pour former un gel qui surnage à la surface du contenu gastrique. Ce gel est capable, grâce au dégagement de $CO_2$, de remonter en cas de reflux et de tapisser la muqueuse essentiellement au niveau cardio-tubérositaire. Il possède une importante viscosité (supérieure à 220 mPa.s) et assure une bonne protection de la muqueuse.

Ce médicament est destiné à être administré au moins une heure après chacun des trois repas.

Concernant le développement galénique, l'objectif fixé était la formation d'un gel surnageant, dit "effet radeau", sur une solution acide.

L'essai "in vitro" consiste à verser un volume de 20 ml de suspension dans une solution d'acide chlorhydrique (150 ml-M/10). Un radeau se forme et flotte à la surface du liquide. Par cet essai sont appréciés :
- la vitesse de formation,
- l'épaisseur,
- la rigidité (résistance à une pénétration manuelle),

- la stabilité (durée de vie du radeau),
- et le gradient de pH entre la surface du gel et l'intérieur du liquide.

**Principes actifs :**

**. Acide alginique :**

Une attention particulière a été apportée à la sédimentation des poudres insolubles dans le produit fini. Elle est directement conditionnée par la viscosité du produit fini et peut donc être modulée par l'emploi d'un acide alginique spécifique.

La viscosité des préparations formées par ce principe actif et donc la stabilité du "radeau", dépend entre autre de la longueur des chaines d'acide uronique qu'il contient.

Un test en éprouvette a orienté nos recherches concernant le choix de l'acide alginique et nous a amené à retenir, en accord avec l'activité pharmacologique (test de l'"effet radeau"), l'acide alginique ayant la viscosité la mieux adaptée à la mise en suspension.

La viscosité retenue permet d'atteindre les caractéristiques évoquées avant, sans empêcher un écoulement aisé du flacon. Elle conditionne également la cohérence, la solidité et la stabilité du radeau (longueur des chaines uroniques).

**. Autres principes Acifs :**

Les principes actifs retenus sont classiquement employés dans la formulation de telles préparations, ils ne présentent aucun caractère galénique particulier. Ils répondent à leur monographie respective et assurent l'activité thérapeutique de la composition selon l'invention ; hydroxyde d'aluminium colloïdal, carbonate de magnésium léger (pouvoir neutralisant) et silice hydratée (pouvoir couvrant).

**Excipient :**

Les excipients associés à ces quatre constituants actifs ont été choisis en raison :
- de leurs propriétés galéniques adaptées à la forme suspension et à l'obtention d'un gel de qualité,
- de leur parfaite innocuité et de leur tolérance,
- de leur radio-transparence.

**. Bicarbonate de sodium :**

Le bicarbonate de sodium intervient dès le contact de la suspension avec le contenu gastrique acide. Il permet, grâce au dégagement d'anhydride carbonique qui se piège dans le gel visqueux, formé par l'acide alginique, d'obtenir une mousse qui monte à la surface du liquide gastrique : le radeau gélifié est formé, responsable des propriétés thérapeutiques de la formulation.

La matière première répond aux normes européennes (Ph. Eur, 2ème édition p. 195), les quantités proposées dans la formule définitive ont été optimisées.

Un résumé de ces différents essais, est présenté dans le tableau suivant. La variable est la concentration en bicarbonate de sodium, la concentration de tous les autres composants étant fixée.

| Concentration en bicarbonate | $<2,5$ % | $2,5$ % | $>2,5$ % |
|---|---|---|---|

**Test radeau :**

| | | | |
|---|---|---|---|
| . rapidité apparition | → | | ↗ |
| . épaisseur | → | 3,5 cm | max. 3,5 cm |
| . gradient | 4 unités pH | 4 unités pH | ↘ |
| . rigidité | ←-------------- | équivalente | -------------→ |
| . stabilité | ←------------- | équivalente | -------------→ |

La concentration de 2,5 % en bicarbonate de sodium a été retenue. Elle apparaît comme étant le meilleur compromis entre les différents caractères recherchés :

. En effet, à des concentrations plus faibles, le gel ne répond pas au test de l'"effet radeau" de façon optimale (rapidité d'apparition, épaisseur,...),

. Le bicarbonate participe à la neutralisation de l'acide chlorhydrique. Mais son rôle est limité ici à la production de $CO_2$ nécessaire à l'obtention de l'"effet radeau", ce qui est obtenu avec une concentration de 2,5 %. Des concentrations élevées de cet excipient seraient susceptibles de provoquer un effet rebond caractérisée par une surproduction d'acide accompagnée d'un déséquilibre acido-basique systémique.

Par ailleurs, un test sur le modèle dynamique de l'estomac artificiel a confirmé la formation satisfaisante du gel surnageant sur un "chyme" acide.

**. Carbonate de calcium :**

L'apport d'ions calcium produits par réaction entre le carbonate de calcium et le contenu gastrique, permet la formation d'une mousse de grande stabilité dans l'estomac. Cette stabilité, dans ces conditions, garantit une durée d'action prolongée.

Différents essais avec et sans calcium ont été réalisés, ils nous ont permis d'opter pour la concentration de 1,2 % en Carbonate de calcium.

Les essais menés avec des préparations dépourvues de calcium ont montré un radeau beaucoup moins stable et solide, conformément à la littérature.

**. Alginate de magnésium :**

L'alginate de magnésium, à faible concentration (0,25 % (m/v)), stabilise la suspension et contribue à l'obtention d'une viscosité assurant le maintien en suspension des différentes poudres insolubles durant la répartition et la prise thérapeutique (viscosité supérieure à 220 mPa.s).

**. Hydroxyde de sodium :**

La solution d'hydroxyde de sodium à 30 %, préparée à partir de pastilles, permet d'ajuster le pH de la suspension à une valeur de 8,2 qui assure la conservation du bicarbonate de sodium.

En son absence, une décomposition lente et continue de cet ingrédient est observée, avec perte d'anhydride carbonique.

**. Glycérol :**

La quantité de glycérol adjointe à la préparation participe à l'obtention de la viscosité définitive.
De plus, la glycérol apporte une saveur sucrée de base à la préparation.

**. Saccharine sodique :**

La saccharine sodique complète l'édulcoration. A faible dose, elle n'apporte pas l'arrière goût persistant caractéristique de ce produit.

**. Composition aromatique :**

Les arômes menthe poivrée et caramel, choisis en raison de leur bonne adaptation au pH du milieu, permettent d'obtenir une préparation satisfaisante et appréciée pour ses caractères organoleptiques.

**. Eau purifiée :**

Matière première quantitativement la plus importante dans la préparation, l'eau purifiée répond aux normes de la Pharmacopée Européenne 2$^{\text{ème}}$ Edition (1990 p. 8).
Une norme de contamination microbienne maximale ($10^2$ germes totaux revivifiables par gramme) a été fixée, conformément aux directives de l'USP XXII concernant l'eau de fabrication.
On prépare une composition répondant à la formule suivante :

| | |
|---|---:|
| - acide alginique | 4,000 g |
| - hydroxyde d'aluminium colloïdal | 0,600 g |
| - silice hydratée | 2,600 g |
| - carbonate de magnésium | 0,800 g |
| - alginate de magnésium | 0,250 g |
| - carbonate de calcium | 1,200 g |
| - glycérol | 12,000 g |
| - saccharine sodique | 0,050 g |
| - arôme caramel | 0,010 g |
| - arôme menthe poivrée | 0,0015g |
| - acide sorbique | 0,190 g |
| - parahydroxybenzoate de méthyle sodé | 0,090 g |
| - parahydroxybenzoate de propyle sodé | 0,045 g |
| - bicarbonate de sodium | 2,500 g |
| - eau purifiée qsp | 100 ml |

et en ce qu'elle contient en outre environ 1 g d'hydroxyde de sodium, de manière à amener le pH à une valeur d'environ 8,2.

**EXEMPLE 2 : APPROCHE IN VITRO DE L'APPARITION DU "GRADIENT DE pH" DANS DES CONDITIONS PROCHES DES CONDITIONS THERAPEUTIOUES.**

Les variations du pH de la surface, correspondant à la fraction de liquide susceptible d'être en contact avec les muqueuses gastrique et oesophagienne, et dans le contenu "intragastrique" ont été appréciées en système dynamique, par le modèle de "l'estomac artificiel" (Aliment Pharmacol. Therap, 1988, 2, 461-470).
1. Le modèle original de "l'estomac artificiel" a été modifié par l'addition d'une seconde chaine de pH-métrie . "L'estomac" S1 est représenté par un récipient qui reçoit un flux de sécrétion S2 et dont le contenu est vidangé par le flux S3, ces deux flux étant régis par une pompe péristaltique (P). Le flux de "sécrétion" a été conservé constant à 3 ml/min, il correspond à une sécrétion acide de 18 mmol/h, c'est-à-dire dans les limites de la stimulation acide par la pentagastrine. Les flux de "vidange" ont été variables de 1,5, 3,0 ou de 4,5 ml/min mimant, par rapport aux données de la physiologie, une vidange de la sécrétion gastrique, lente, égale à la sécrétion ou rapide (Gastroenterol. Clin. Biol., 1985, 9, 902-910).

Une électrode combinée (Schott N 62), connectée à un pH-mètre (Schott CG 52) a été placée dans le contenu de S1 et a mesuré les variations de pH de la phase liquide. Une microélectrode combinée (Heito BRV 15 H), connectée à un pH-mètre (Heito PSD 20), placée au-dessous de la surface du contenu de S1, a mesuré le pH par contact en surface du contenu "gastrique". La position de cette électrode a été ajustée manuellement, lorsque les flux de vidange ont été différents du flux de sécrétion. Un index fixé sur l'électrode a permis son positionnement, entre 3 et 5 mm au-dessous de la surface. Les deux pH-mètres reliés à un enregistreur à deux canaux (Servodor SE 120) ont mesuré l'évolution du pH depuis l'introduction des solutions à tester aux deux niveaux du contenu "gastrique" jusqu'au retour au pH initial de S1.

Avant chaque essai, les pH-mètres et les deux canaux de l'enregistreur ont été étalonnés avec des solutions tampons de pH calibrés.

2. Pour simuler les conditions physicochimiques "intragastrique", les expérimentations ont été conduites avec un "pool" de suc gastrique humain afin de reproduire les conditions réelles d'activité de ces médicaments ou avec des solutions aqueuses d'acide chlorhydrique pour l'analyse de leur comportement pharmacologique.

a) S1 contient 100 ml de suc gastrique humain (116 mmol/l, pH 0,80) qui représente également la "sécrétion" S2 (1 essai par flux de vidange et par médicament).

b) S1 contient 100 ml d'HCl 0,1 N, cette solution constituant le flux de "sécrétion" S2 (2 essais pour les flux de vidange 3 et 4,5 ml/min et l'essai en réponse au flux de 1,5 ml/min et par médicament).

c) S1 contient 100 ml d'HCl O,1 N additionné d'extrait de viande à la concentration finale de 1 %, le flux de "sécrétion" S2 étant représenté par HCl O,1 N (2 essais pour les flux de vidange de 3 et 4,5 ml/min et 1 essai en réponse au flux de vidange de 1,5 ml/min et par médicament).

3. Après que S1 ait été rempli de la solution expérimentale choisie, la "sécrétion" et la "vidange" simulées ont été mises en oeuvre. Le pH, au sein de S1 et à sa surface, a été enregistré de façon continue jusqu'au retour au pH initial du "contenu gastrique".

4. Les paramètres mesurés ont été :

a) <u>entre 3 et 5 mm au-dessous de la surface</u>, les valeurs de pH ont été mesurées pendant toute la durée de l'essai.

b) <u>dans le "contenu gastrique"</u> : -i/ le pH maximum et la quantité d'acide consommée (mmol H+) pour retrouver le pH initial, ce calcul tenant compte de la quantité d'acide dans S1 au temps 0 et de la quantité d'acide infusée. -ii/ la caractérisation et la contribution relative de l'activité de neutralisation et de la capacité tampon dans le mécanisme de l'antiacidité.

Ce dispositif a permis d'évaluer la capacité antiacide et d'apprécier l'existence d'un gradient de pH, en réponse aux modifications permanentes de la concentration acide et de la concentration en médicament.

L'activité antiacide et la capacité d'induction d'un gradient de pH de la composition selon l'invention ont été mesurées par comparaison avec les produits suivants :

GAVISCON® Suspension

ALGICON® Suspension

GAVISCON® suspensions contient, pour 10 ml de suspension :

| - Alginate de sodium : | 500 mg |
| --- | --- |
| - Bicarbonate de sodium : | 267 mg |

ALGICON® suspension contient, pour 10 ml de suspension :

| - gel d'hydroxyde d' aluminium et de carbonate de magnésium | 280 mg |
| --- | --- |
| - carbonate de magnésium léger | 350 mg |
| - alginate de magnésium | 500 mg |
| - carbonate de calcium | 150 mg |
| - carbonate acide de potassium | 100 mg |

Les prises d'essai de chacun des médicaments correspondant à l'unité thérapeutique recommandée par les fabricants.

## RESULTATS

## 1. CAPACITE ANTIACIDE.

### a) Addition à 100 ml de suc gastrique humain (116 mmol/l. pH 0,80).

Les trois substances testées additionnées à 100 ml de suc gastrique humain exercent une activité antiacide, caractérisée par l'élévation du pH et les longs délais nécessaires pour retrouver pH 1,0.

Le tableau I groupe le résultat des essais : pH maximum, délais nécessaires pour atteindre les pH de référence et nombre de millimoles d'acide consommées pour retrouver le pH initial (pH 0,80). A noter que les tests avec le flux de vidange 1,5 ml/min n'ont pu être poursuivis au-delà de 40 min, par manque de suc gastrique.

## TABLEAU I :

### COMPOSITION SELON L'INVENTION

| Vidange gastrique | | | |
|---|---|---|---|
| (ml/min) | 1,5 | 3,0 | 4,5 |
| pH maximum | 1,65 | 1,75 | 2,15 |
| Temps (min) pour atteindre | | | |
| pH 1,5 | 12 | 5 | 8 |
| pH 1,2 | 18 | 14 | 10 |
| pH 1,0 | 38 | 29 | 22 |
| pH 0,80 | - | 125 | 65 |

Millimoles d'acide consommées pour retrouver pH 0,80

55,1    34,2

### GAVISCON SUSPENSION

| Vidange gastrique | | | |
|---|---|---|---|
| (ml/min) | 1,5 | 3,0 | 4,5 |
| pH maximum | 1,50 | 1,40 | 1,50 |
| Temps (min) pour atteindre | | | |
| pH 1,2 | 17 | 9 | 6 |
| pH 1,0 | 41 | 21 | 13 |
| pH 0,80 | - | 92 | 62 |

Millimoles d'acide consommées pour retrouver pH 0,80

43,6       33,1

10

## ALGICON SUSPENSION

| Vidange gastrique | | | |
|---|---|---|---|
| (ml/min) | 1,5 | 3,0 | 4,5 |
| pH maximum | 3,0 | 2,70 | 5,30 |
| Temps (min) pour atteindre | | | |
| pH 2,0 | 7 | 3 | 19 |
| pH 1,5 | 21 | 11 | 22 |
| pH 1,2 | 35 | 21 | 30 |
| pH 1,0 | - | 38 | 38 |
| pH 0,80 | - | 115 | 60 |

Millimoles d'acide consommées pour retrouver pH 0,80

| | 51,6 | 32,4 |
|---|---|---|

**b) Addition à 100 ml d'HCl 0,1 N.**

Le tableau II groupe les résultats moyens des essais : pH maximum, les délais nécessaires pour atteindre les pH de référence et le nombre de millimoles d'acide consommées pour retrouver pH 1,0.

## TABLEAU II

## COMPOSITION SELON L'INVENTION

| Vidange gastrique | | | |
|---|---|---|---|
| (ml/min) | 1,5 | 3,0 | 4,5 |
| pH maximum | 5,30 | 5,40 | 2,10 |
| Temps (min) pour atteindre | | | |
| pH 2,0 | 11 | 14,5 | 10 |
| pH 1,5 | 25 | 28,5 | 8,5 |
| pH 1,2 | 58 | 55 | 16,5 |
| pH 1,0 | 150 | 115 | 41,5 |

Millimoles d'acide consommées pour atteindre pH 1,0

| 55 | 44,5 | 22,4 |
|---|---|---|

11

Relation entre la résistance induite au retour à pH 1,0 (mmol H+) et le rapport entre les flux de sécrétion (3,0 ml/min) et de vidange (1,5, 3,0 et 4,5 ml/min), soit 2, 1, 0,66

$$y = 21,19 \ x + 14,7 \ (r=0,887)$$

## GAVISCON SUSPENSION

| Vidange gastrique (ml/min) | 1,5 | 3,0 | 4,5 |
|---|---|---|---|
| pH maximum | 4,0 | 2,95 | 5,42 |
| Temps (min) pour atteindre | | | |
| pH 2,0 | 7 | 4,5 | 12,5 |
| pH 1,5 | 16 | 13 | 18 |
| pH 1,2 | 38 | 32,5 | 34,5 |
| pH 1,0 | 95 | 80 | 60 |

Millimoles d'acide consommées pour atteindre pH 1,0

| 38,5 | 34 | 28 |
|---|---|---|

Relation entre la résistance induite au retour à pH 1,0 (mmol H+) et le rapport entre les flux de sécrétion et de vidange, soit 2, 1, 0,66

$$y = 7,07 \ x + 24,8 \ (r = 0,936)$$

## ALGICON SUSPENSION

| Vidange gastrique (ml/min) | 1,5 | 3,0 | 4,5 |
|---|---|---|---|
| pH maximum | 5,5 | 5,6 | 5,75 |
| Temps (min) pour atteindre | | | |
| pH 2,0 | 21 | 25 | 24 |
| pH 1,5 | 36 | 34 | 34 |
| pH 1,2 | 83 | 55 | 49 |
| pH 1,0 | 140 | 97,5 | 65 |

Millimoles d'acide consommées pour atteindre pH 1,0

<div align="center">

52      39,2   29,5

</div>

Relations entre la résistance induite au retour à pH 1,0 (mmol H+) et le rapport entre les flux de sécrétion et de vidange, soit 2, 1, 0,66

$$y = 15{,}88\ x + 20{,}8\quad (r=0{,}980)$$

**c) Addition à 100 ml d'HCl 0,1 N enrichi en extrait de vidange à la concentration finale de 1 %.**

Le tableau III groupe les résultats moyens des essais : pH maximum, les délais nécessaires pour atteindre les pH de référence et le nombre de millimoles d'acide consommées pour retrouver pH 1,0.

## TABLEAU III

### COMPOSITION SELON L'INVENTION

| Vidange gastrique | | | |
|---|---|---|---|
| (ml/min) | 1,5 | 3,0 | 4,5 |
| pH maximum | 5,10 | 5,75 | 5,40 |
| Temps (min) pour atteindre | | | |
| pH 2,0 | 35 | 34,5 | 19 |
| pH 1,5 | 53 | 46 | 25,5 |
| pH 1,2 | 90 | 68 | 36 |
| pH 1,0 | 170 | 130 | 49 |

Millimoles d'acide consommées pour atteindre pH 1,0

<div align="center">

61      49   24,7

</div>

Relation entre la résistance induite au retour à pH 1,0 (mmol H+) et le rapport entre les flux de sécrétion et de vidange, soit 2, 1, 0,66

$$y = 23{,}66\ x + 16{,}02\quad (r = 0{,}891)$$

EP 0 506 563 B1

## GAVISCON SUSPENSION

Vidange gastrique

    (ml/min)            1,5        3,0    4,5

pH maximum         4,90      4,10   5,25

Temps (min) pour atteindre

    pH 2,0         20        14,5   21

    pH 1,5         31        20,5   29,5

    pH 1,2         53        36,5   44,5

    pH 1,0         90        72,5   60


Millimoles d'acide consommées pour atteindre pH 1,0

                         37        31,7   28

Relation entre la résistance induite au retour à pH 1,0 (mmol H+) et le rapport entre les flux de sécrétion et de vidange, soit 2, 1, 0,66

$$y = 6,39\ x + 24,4\ (r = 0,984)$$

## ALGICON SUSPENSION

Vidange gastrique

    (ml/min)            1,5        3,0    4,5

pH maximum         5,5       5,5    5,5

Temps (min) pour atteindre

    pH 2,0         53        30,5   31,5

    pH 1,5         76        41     40

    pH 1,2         120       57     49,5

    pH 1,0         180       85     65


Millimoles d'acide consommées pour atteindre pH 1,0

                         64        35,5    29,5


Relation entre la résistance induite au retour à pH 1,0 (mmol H+) et le rapport entre les flux de sécrétion et de vidange, soit 2, 1, 0,66

$$y = 26,71\ x + 9,91\ (r = 0,992)$$

14

## 2. pH AU NIVEAU DE LA SURFACE

### a) Les médicaments sont additionnés à 100 ml de suc gastrique (116 mmol/l. pH 0,80).

Le pH moyen en surface figure dans le Tableau IV.

Tableau IV

| Vidange gastrique (ml/min) | 1,5 | 3,0 | 4,5 |
|---|---|---|---|
| Composition selon l'invention | 3,52 | 4,19 | 2,90 |
| Gaviscon Suspension | 2,27 | 2,09 | 2,00 |
| Algicon Suspension | 5,18 | 4,51 | 5,44 |

Les niveaux de pH les plus élevés sont trouvés avec Algicon Suspension quelque soit le flux de vidange, compris entre 4,51 et 5,44 puis avec la composition selon l'invention avec des pH compris entre 2,9 et 4,19, dépendant de la vidange. Enfin, Gaviscon Suspension induit des niveaux de pH en surface compris entre 2,0 et 2,27. Par rapport au liquide gastrique testé, il apparaît dans tous les cas qu'un gradient puissant s'installe puisque pour une concentration acide intragastrique de 116 mmol/ l, le pH de la surface indique une concentration comprise entre 10 mmol/l, au plus bas à 0,01 mmol/l selon les préparations.

### b) Les médicaments sont additionnés à 100 ml d'HCl 0,1 N.

Le pH moyen en surface figure dans le tableau V. Les valeurs sont calculées à partir des valeurs moyennes des 2 essais en réponse aux flux de vidange 3,0 et 4,5 ml/min.

Tableau V

| Vidange gastrique (ml/min) | 1,5 | 3,0 | 4,5 |
|---|---|---|---|
| Composition selon l'invention | 4,13 + - 0,52 | 6,15 + - 0,30 | 6,31 + - 0,05 |
| Gaviscon Suspension | 3,83 + - 0,36 | 4,72 + - 0,12 | 5,28 + - 0,16 |
| Algicon Suspension | 6,78 + - 0,20 | 6,49 + - 0,05 | 6,71 + - 0,04 |

Les niveaux de pH les plus élevés sont trouvés avec Algicon Suspension quelque soit le flux de vidange, compris entre 6,49 et 6,78, puis avec la composition selon l'invention, dans la même échelle de pH pour les flux de vidange 3,0 et 4,5 ml/min mais à un niveau plus bas en réponse à la vidange lente. Gaviscon Suspension induit des niveaux de pH en surface, croissant avec l'accroissement des flux de vidange, compris entre 3,83 et 5,28.

### c) Les médicaments sont additionnés à 100 ml d'HCl 0,1 N enrichi en extrait de viande à la concentration finale de 1%.

Le Tableau VI groupe les valeurs moyennes des pH.

Tableau VI

| Vidange gastrique (ml/min) | 1,5 | 3,0 | 4,5 |
|---|---|---|---|
| Composition selon l'invention | 5,55 + - 0,20 | 6,32 + - 0,03 | 5,33 + - 0,09 |
| Gaviscon Suspension | 4,61 + - 0,14 | 5,33 + - 0,02 | 5,20 + - 0,14 |
| Algicon Suspension | 4,91 + - 0,77 | 6,82 + - 0,18 | 6,85 + - 0,19 |

Les niveaux de pH observés sont plus élevés en présence de protéines avec les suspensions de composition selon l'invention, Gaviscon et Algicon pour l'ensemble des flux de vidange par rapport aux niveaux de pH observés en présence d'HCl 0,1 N sans protéines. Cette élevation est plus nette en réponse à la vidange de 1,5 ml/min mais elle tend à s'effacer pour les autres flux de vidange.

## 3. GRADIENT DE pH ENTRE LA SURFACE ET LE "CONTENU GASTRIOUE".

### a) Dans 100 ml de suc gastrique humain.

En présence d'un pool de suc gastrique humain acide de concentration égale à 116 mmol/l et de pH 0,80, la composition selon l'invention induit une différence de pH qui s'instaure immédiatement et est variable selon le flux de vidange. Après 30 minutes, le pH intragastrique est compris entre 1 et 0,80 et le pH en surface est compris entre 3,8, 4,0 et 2,5 en réponse aux flux de vidange 1,5, 3 et 4,5 ml/min. Lorsque le pH intragastrique retrouve le pH initial, le pH de la surface est compris entre 2 et 4, ce qui représente une différence de concentration acide de 116 mmol/l versus 10 et 0,1 mmol/l.

Gaviscon Suspension induit un gradient de plus faible intensité quelque soit le flux de vidange, puisque le pH de la surface reste proche de 2,0 tandis que le contenu gastrique atteint pH 0,80. Sur le plan des concentrations, la surface contient environ 10 mmol/l alors que le liquide gastrique atteint 116 mmol/l/

Algicon Suspension, au contraire induit un gradient de pH légèrement plus faible que celui observé en milieu HCl 0,1 N, puisque le pH en surface est compris entre 0,4 et 6,0 mais n'est pas supérieur à 6,0. Le gradient est indépendant des flux de vidange et de la durée des tests. La différence de concentrations entre le contenu gastrique et sa surface est de 116 à 0,01 mmol/l d'acide.

### b) Dans 100 ml d'HCl 0,1 N :

La composition selon l'invention induit une différence de pH croissante avec le temps entre le contenu gastrique et sa surface, en réponse au flux de vidange de 3 ml/min : le pH est peu différent entre les 2 phases à t 0 mais le gradient s'établit rapidement de telle sorte qu'àprès 30 min le pH intragastrique est de l'ordre de 1,5 alors qu'en surface il est supérieur à 6,0.

En réponse au flux de vidange de 1,5 ml/min, le pH est peu différent entre les 2 phases ; il décroit ensuite dans le contenu gastrique alors qu'il persiste vers pH 5,0 jusqu'à la 40ème minute, puis il décroit avec l'acroissement de la durée du test jusqu'à 2,8 alors que le pH du contenu gastrique retrouve sa valeur initiale.

En réponse au flux de vidange, 4,5 ml/min le pH de la surface reste constant vers pH 6,0 alors que le pH intragastrique décroit de 3 à 1. Dans tous les cas, le gradient de pH atteint une amplitude d'au moins 4 unités de pH. Avec la vidange lente, une perméabilisation de la surface au liquide acide est perceptible et le gradient est plus faible, inférieur à 2 unités de pH.

Gaviscon Suspension induit une différence de pH comprise entre 3 et 4 unités de pH selon les conditions expérimentales. En réponse aux flux de vidange de 3 et 4,5 ml/min le pH de la surface est proche de 5,0. Avec la vidange lente, le pH en surface atteint 5,0 dans les premières minutes puis il décroit dans le temps par suite d'une perméabilisation de la surface au liquide acide jusqu'à pH 3,0. Le gradient est alors plus faible de 2 unités de pH seulement.

Algicon Suspension induit un gradient de même intensité quelques soient les flux de vidange, puisque le pH en surface est toujours supérieur à 6,0, alors que le pH du "contenu intragastrique" décroit vers pH 1,0.

### c) Les médicaments sont additionnés à 100 ml d'HCl 0,1 N enrichi en extrait de viande à la concentration finale de 1 %.

Composition selon l'invention : tandis que le pH intragastrique est plus élevé qu'en l'absence de protéines, le pH en surface atteint les mêmes niveaux, soit entre pH 5,0 et 6,0 pour les flux de vidange 3 et 4,5 ml/Min. En réponse à la vidange lente, le pH de surface reste constant compris entre pH 5,5 et pH 6,0 ; il n'y a pas de perméabilisation au contenu acide comme cela a été objectivé en l'absence de protéines, la différence de pH entre les deux phases est comprise entre 4 et 5 unités de pH.

Gaviscon Suspension induit sensiblement le même gradient qu'en l'absence de protéines de l'ordre de 3,5 à 4 unités de pH.

Algicon Suspension induit un gradient entre les 2 phases sensiblement identique à celui induit en l'absence de protéines puisque le pH en surface est de l'ordre de 7,0 alors que le pH du "contenu intragastrique" décroit de 5,0 à 1,0. A la 30ème minute l'intensité du gradient est de 5 unités de pH pour les flux de vidange 3 et 4,5 ml/min et plus faible, de 3,5 unités de pH avec la vidange lente qui paraît induire une perméabilisation de la surface au contenu acide.

**Revendications**

**Revendications pour les Etats contractants suivants : AT, BE, CH, LI, DE, DK, FR, GB, IT, LU, MC, NL, PT, SE**

1. Composition pharmaceutique sous forme de suspension, caractérisée en ce qu'elle contient de l'acide alginique à des concentrations comprises entre 2 et 7 % (p/v), du bicarbonate de sodium à des concentrations comprises entre 1,5 et 7 % (p/v) et au moins un agent viscosant choisi dans le groupe comprenant la gomme xanthane, l'alginate de magnésium, et le glycérol.

2. Composition pharmaceutique, selon la revendication 1, caractérisée en ce qu'elle comporte à titre de principe actif de l'acide alginique, de l'hydroxyde d'aluminium colloïdal, du carbonate de magnésium, et de la silice hydratée, et en ce qu'elle comporte en outre une faible proportion d'au moins un agent viscosant choisi dans le groupe comprenant la gomme xanthane, l'alginate de magnésium, et le glycérol.

3. Composition pharmaceutique selon l'une des revendications 1 et 2, caractérisée en ce qu'elle contient à titre d'agent viscosant, de l'alginate de magnésium, dans une proportion comprise entre 0,1 et 2 % (p/v).

4. Composition pharmaceutique selon l'une des revendications 1 à 3, caractérisée en ce qu'elle contient de l'alginate de magnésium dans une proportion au plus égale à environ 0,25 % (p/v).

5. Composition pharmaceutique selon l'une des revendications 1 à 4, caractérisée en ce qu'elle contient du bicarbonate de sodium, dans une proportion de 2,5 % (p/v).

6. Composition pharmaceutique selon l'une des revendications 1 à 5, caractérisée en ce qu'une unité de dosage de 10 ml de suspension contient :

| | |
|---|---|
| - acide alginique | 0,40 g |
| - hydroxyde d'aluminium colloïdal | 0,06 g |
| - carbonate de magnésium | 0,08 g et |
| - silice précipitée | 0,26 g |

7. Composition pharmaceutique selon l'une des revendications 1 à 6, caractérisée en ce qu'elle contient en outre des agents édulcorants, aromatisants, et des conservateurs pharmaceutiquement acceptables.

8. Composition pharmaceutique selon l'une des revendications 1 à 7, caractérisée en ce qu'elle répond à la formule suivante :

| | |
|---|---|
| - acide alginique | 4,000 g |
| - hydroxyde d'aluminium colloïdal | 0,600 g |
| - silice hydratée | 2,600 g |
| - carbonate de magnésium | 0,800 g |
| - alginate de magnésium | 0,250 g |
| - carbonate de calcium | 1,200 g |
| - glycérol | 12,000 g |
| - saccharine sodique | 0,050 g |
| - arôme caramel | 0,010 g |
| - arôme menthe poivrée | 0,0015g |
| - acide sorbique | 0,190 g |
| - parahydroxybenzoate de méthyle sodé | 0,090 g |
| - parahydroxybenzoate de propyle sodé | 0,045 g |
| - bicarbonate de sodium | 2,500 g |
| - eau purifiée qsp | 100 ml |

et en ce qu'elle contient en outre environ 1 g d'hydroxyde de sodium, de manière à amener le pH à une valeur d'environ 8,2.

**Revendications pour les Etats contractants suivants : ES, GR**

1. Procédé de fabrication d'une composition pharmaceutique sous forme de suspension, caractérisé en ce que l'on mélange de l'acide alginique à des concentrations comprises entre 2 et 7 % (p/v), du bicarbonate de sodium à des concentrations comprises entre 1,5 et 7% (p/v) et au moins un agent viscosant choisi dans le groupe comprenant la gomme xanthane, l'alginate de magnésium et le glycérol.

2. Procédé selon la revendication 1, caractérisé en ce que l'on utilise à titre de principe actif de l'acide alginique, de l'hydroxyde d'aluminium colloïdal, du carbonate de magnésium, et de la silice hydratée, et en ce que l'on utilise en outre une faible proportion d'au moins un agent viscosant choisi dans le groupe comprenant la gomme xanthane, l'alginate de magnésium, et le glycérol.

3. Procédé selon l'une des revendications 1 et 2, caractérisé en ce que l'on utilise à titre d'agent viscosant, de l'alginate de magnésium, dans une proportion comprise entre 0,1 et 2 % (p/v).

4. Procédé selon l'une des revendications 1 à 3, caractérisé en ce que l'on utilise de l'alginate de magnésium dans une proportion au plus égale à environ 0,25 % (p/v).

5. Procédé selon l'une des revendications 1 à 4, caractérisé en ce que l'on utilise du bicarbonate de sodium, dans une proportion de 2,5 % (p/v).

6. Procédé selon l'une des revendications 1 à 5, caractérisé en ce que l'on prépare une unité de dosage de 10 ml de suspension contenant :

| | |
|---|---|
| - acide alginique | 0,40 g |
| - hydroxyde d'aluminium colloïdal | 0,06 g |
| - carbonate de magnésium | 0,08 g et |
| - silice précipitée | 0,26 g |

7. Procédé selon l'une des revendications 1 à 6, caractérisé en ce que l'on ajoute en outre des agents édulcorants, aromatisants, et des conservateurs pharmaceutiquement acceptables.

8. Procédé selon l'une des revendications 1 à 7, caractérisé en ce que l'on prépare une composition répondant à la formule suivante :

| | |
|---|---|
| - acide alginique | 4,000 g |
| - hydroxyde d'aluminium colloïdal | 0,600 g |
| - carbonate de magnésium | 0,800 g |
| - silice hydratée | 2,600 g |
| - alginate de magnésium | 0,250 g |
| - carbonate de calcium | 1,200 g |
| - glycérol | 12,000 g |
| - saccharine sodique | 0,050 g |
| - arôme caramel | 0,010 g |
| - arôme menthe poivrée | 0,0015 g |
| - acide sorbique | 0,190 g |
| - parahydroxybenzoate de méthyle sodé | 0,090 g |
| - parahydroxybenzoate de propyle sodé | 0,045 g |
| - bicarbonate de sodium | 2,500 g |
| - eau purifiée qsp | 100 ml |

à laquelle on ajoute en outre environ 1 g d'hydroxyde de sodium, de manière à amener le pH à une valeur d'environ 8,2.

**Claims**
**Claims for the following Contracting States : AT, BE, CH, LI, DE, DK, FR, GB, IT, LU, MC, NL, PT, SE**

1. Pharmaceutical composition in suspension form, characterized in that in contains alginic acid at concentrations of between 2 and 7 % (w/v), sodium bicarbonate at concentrations of between 1.5 and 7 % (w/v) and at least one viscosity control agent chosen from the group comprising xanthan gum, magnesium alginate and glycerol.

2. Pharmaceutical composition according to Claim 1, characterized in that it contains as active principle alginic acid, colloidal aluminium hydroxide, magnesium carbonate and hydrated silica, and in that it contains, in addition, a small proportion of at least one viscosity control agent chosen from the group comprising xanthan gum, magnesium alginate and glycerol.

3. Pharmaceutical composition according to either of Claims 1 and 2, characterized in that it contains as viscosity control agent magnesium alginate, in a proportion of between 0.1 and 2 % (w/v).

4. Pharmaceutical composition according to one of Claims 1 to 3, characterized in that it contains magnesium alginate in a proportion equal to not more than approximately 0.25 % (w/v).

5. Pharmaceutical composition according to one of Claims 1 to 4, characterized in that it contains sodium bicarbonate in a proportion of 2.5 % (w/v).

6. Pharmaceutical composition according to one of Claims 1 to 5, characterized in that a dosage unit of 10 ml of suspension contains:

| | |
|---|---|
| - alginic acid | 0.40 g |
| - colloidal aluminium hydroxide | 0.06 g |
| - magnesium carbonate | 0.08 g and |
| - precipitated silica | 0.26 g |

7. Pharmaceutical composition according to one of Claims 1 to 6, characterized in that it contains, in addition, sweetening and flavouring agents and pharmaceutically acceptable preservatives.

8. Pharmaceutical composition according to one of Claims 1 to 7, characterized in that it corresponds to the following formula:

| | |
|---|---|
| - alginic acid | 4.000 g |
| - colloidal aluminium hydroxide | 0.600 g |
| - hydrated silica | 2.600 g |
| - magnesium carbonate | 0.800 g |
| - magnesium alginate | 0.250 g |
| - calcium carbonate | 1.200 g |
| - glycerol | 12.000 g |
| - saccharin sodium | 0.050 g |
| - caramel flavouring | 0.010 g |
| - peppermint flavouring | 0.0015 g |
| - sorbic acid | 0.190 g |
| - sodium methyl para-hydroxybenzoate | 0.090 g |
| - sodium propyl para-hydroxybenzoate | 0.045 g |
| - sodium bicarbonate | 2.500 g |
| - purified water qs | 100 ml |

and in that it contains, in addition, approximately 1 g of sodium hydroxide so as to bring the pH to a value of approximately 8.2.

**Claims for the following Contracting States : ES, GR**

1.  Process for the manufacture of a pharmaceutical composition in suspension form, characterized in that alginic acid at concentrations of between 2 and 7 % (w/v), sodium bicarbonate at concentrations of between 1.5 and 7 % (w/v) and at least one viscosity control agent chosen from the group comprising xanthan gum, magnesium alginate and glycerol are mixed.

2.  Process according to Claim 1, characterized in that alginic acid, colloidal aluminium hydroxide, magnesium carbonate and hydrated silica are used as active principle, and in that a small proportion of at least one viscosity control agent chosen from the group comprising xanthan gum, magnesium alginate and glycerol is used in addition.

3.  Process according to one of Claims 1 and 2, characterized in that magnesium alginate, in a proportion of between 0.1 and 2 % (w/v), is used as viscosity control agent.

4.  Process according to one of Claims 1 to 3, characterized in that magnesium alginate is used in a proportion equal to not more than approximately 0.25 % (w/v).

5.  Process according to one of Claims 1 to 4, characterized in that sodium bicarbonate is used in a proportion of 2.5 % (w/v).

6.  Process according to one of Claims 1 to 5, characterized in that a dosage unit of 10 ml of suspension containing the following is prepared:

| | |
|---|---|
| - alginic acid | 0.40 g |
| - colloidal aluminium hydroxide | 0.06 g |
| - magnesium carbonate | 0.08 g and |
| - precipitated silica | 0.26 g |

7.  Process according to one of Claims 1 to 6, characterized in that sweetening and flavouring agents and pharmaceutically acceptable preservatives are added in addition.

8.  Process according to one of Claims 1 to 7, characterized in that a composition corresponding to the following formula is prepared:

20

| | |
|---|---|
| - alginic acid | 4.000 g |
| - colloidal aluminium hydroxide | 0.600 g |
| - magnesium carbonate | 0.800 g |
| - hydrated silica | 2.600 g |
| - magnesium alginate | 0.250 g |
| - calcium carbonate | 1.200 g |
| - glycerol | 12.000 g |
| - saccharin sodium | 0.050 g |
| - caramel flavouring | 0.010 g |
| - peppermint flavouring | 0.0015 g |
| - sorbic acid | 0.190 g |
| - sodium methyl para-hydroxybenzoate | 0.090 g |
| - sodium propyl para-hydroxybenzoate | 0.045 g |
| - sodium bicarbonate | 2.500 g |
| - purified water qs | 100 ml |

to which formula approximately 1 g of sodium hydroxide is added in addition so as to bring the pH to a value of approximately 8.2.

**Patentansprüche**
**Patentansprüche für folgende Vertragsstaaten : AT, BE, CH, LI, DE, DK, FR, GB, IT, LU, MC, NL, PT, SE**

1. Pharmazeutische Zusammensetzung in Form einer Suspension, dadurch gekennzeichnet, daß sie enthält Alginsäure in Konzentrationen zwischen 2 und 7 Gew./Vol.-%, Natriumbicarbonat in Konzentrationen zwischen 1,5 und 7 Gew./Vol.-% und mindestens ein Viskosität verleihendes Mittel (Verdickungsmittel), ausgewählt aus der Gruppe Xanthangummi, Magnesiumalginat und Glycerin.

2. Pharmazeutische Zusammensetzung nach Anspruch 1, dadurch gekennzeichnet, daß sie als aktives Prinzip (Wirkstoff) Alginsäure, kolloidales Aluminiumhydroxid, Magnesiumcarbonat und hydratisiertes Siliciumdioxid enthält und daß sie außerdem einen geringen Mengenanteil mindestens eines Verdickkungsmittels, ausgewählt aus der Gruppe Xanthangummi, Magnesiumalginat und Glycerin, enthält.

3. Pharmazeutische Zusammensetzung nach einem der Ansprüche 1 und 2, dadurch gekennzeichnet, daß sie als Verdickungsmittel Magnesiumalginat in einem Mengenanteil zwischen 0,1 und 2 Gew./Vol.-% enthält.

4. Pharmazeutische Zusammensetzung nach einem der Ansprüche 1 bis 3, dadurch gekennzeichnet, daß sie Magnesiumalginat in einem Mengenanteil von höchstens etwa 0,25 Gew./Vol.-% enthält.

5. Pharmazeutische Zusammensetzung nach einem der Ansprüche 1 bis 4, dadurch gekennzeichnet, daß sie Natriumbicarbonat in einem Mengenanteil von 2,5 Gew./Vol.-% enthält.

6. Pharmazeutische Zusammensetzung nach einem der Ansprüche 1 bis 5, dadurch gekennzeichnet, daß eine Dosiseinheit von 10 ml Suspension enthält:

| | |
|---|---|
| Alginsäure | 0,40 g |
| kolloidales Aluminiumhydroxid | 0,06 g |
| Magnesiumcarbonat | 0,08 g und |
| ausgefälltes Siliciumdioxid | 0,26 g. |

7. Pharmazeutische Zusammensetzung nach einem der Ansprüche 1 bis 6, dadurch gekennzeichnet, daß sie außerdem pharmazeutisch akzeptable Süßungsmittel, Aromatisierungsmittel und Konservierungsmittel enthält.

8. Pharmazeutische Zusammensetzung nach einem der Ansprüche 1 bis 7, dadurch gekennzeichnet, daß sie die folgende Zusammensetzung hat:

| | |
|---|---|
| Alginsäure | 4,000 g |
| kolloidales Aluminiumhydroxid | 0,600 g |
| hydratisiertes Siliciumdioxid | 2,600 g |
| Magnesiumcarbonat | 0,800 g |
| Magnesiumalginat | 0,250 g |
| Calciumcarbonat | 1,200 g |
| Glycerin | 12,000 g |
| Natriumsaccharin | 0,050 g |
| Caramel-Aroma | 0,010 g |
| Pfefferminz-Aroma | 0,0015 g |
| Sorbinsäure | 0,190 g |
| Methylnatrium-p-hydroxybenzoat | 0,090 g |
| Propylnatrium-p-hydroxybenzoat | 0,045 g |
| Natriumbicarbonat | 2,500 g |
| gereinigtes Wasser | ad    100 ml |

und daß sie außerdem etwa 1 g Natriumhydroxid enthält, um den pH-Wert auf einen Wert von etwa 8,2 zu bringen.

**Patentansprüche für folgende Vertragsstaaten : ES, GR**

1. Verfahren zur Herstellung einer pharmazeutischen Zusammensetzung in Form einer Suspension, dadurch gekennzeichnet, daß man Alginsäure in Konzentrationen zwischen 2 und 7 Gew./Vol.-%, Natriumbicarbonat in Konzentrationen zwischen 1,5 und 7 Gew./Vol.-% und mindestens ein Viskosität verleihendes Mittel (Verdickungsmittel), ausgewählt aus der Gruppe Xanthangummi, Magnesiumalginat und Glycerin, miteinander mischt.

2. Verfahren an nach Anspruch 1, dadurch gekennzeichnet, daß man als aktives Prinzip (Wirkstoff) Alginsäure, kolloidales Aluminiumhydroxid, Magnesiumcarbonat, hydratisiertes Siliciumdioxid verwendet und daß man außerdem einen geringen Mengenanteil mindestens eines Verdickungsmittels, ausgewählt aus der Gruppe Xanthangummi, Magnesiumalginat und Glycerin, verwendet.

3. Verfahren nach einem der Ansprüche 1 und 2, dadurch gekennzeichnet, daß man als Verdickungsmittel Magnesiumalginat in einem Mengenanteil zwischen 0,1 und 2 Gew./Vol.-% verwendet.

4. Verfahren nach einem der Ansprüche 1 bis 3, dadurch gekennzeichnet, daß man Magnesiumalginat in einem Mengenanteil von höchstens etwa 0,25 Gew./Vol.-% verwendet.

5. Verfahren nach einem der Ansprüche 1 bis 4, dadurch gekennzeichnet, daß man Natriumbicarbonat in einem Mengenanteil von 2,5 Gew./Vol.-% verwendet.

6. Verfahren nach einem der Ansprüche 1 bis 5, dadurch gekennzeichnet, daß man eine Dosiseinheit von 10 ml Suspension herstellt, die enthält:

| | |
|---|---|
| Alginsäure | 0,40 g |
| kolloidales Aluminiumhydroxid | 0,06 g |
| Magnesiumcarbonat | 0,08 g und |
| ausgefälltes Siliciumdioxid | 0,26 g. |

7. Verfahren nach einem der Ansprüche 1 bis 6, dadurch gekennzeichnet, daß man außerdem pharmazeutisch akzeptable Süßungsmittel, Aromatisierungsmittel und Konservierungsmittel zusetzt.

22

**8.** Verfahren nach einem der Ansprüche 1 bis 7, dadurch gekennzeichnet, daß man eine Zusammensetzung herstellt, welche die folgende Zusammensetzung hat:

| | | |
|---|---|---|
| Alginsäure | | 4,000 g |
| kolloidales Aluminiumhydroxid | | 0,600 g |
| Magnesiumcarbonat | | 0,800 g |
| hydratisiertes Siliciumdioxid | | 2,600 g |
| Magnesiumalginat | | 0,250 g |
| Calciumcarbonat | | 1,200 g |
| Glycerin | | 12,000 g |
| Natriumsaccharin | | 0,050 g |
| Caramel-Aroma | | 0,010 g |
| Pfefferminz-Aroma | | 0,0015 g |
| Sorbinsäure | | 0,190 g |
| Methylnatrium-p-hydroxybenzoat | | 0,090 g |
| Propylnatrium-p-hydroxybenzoat | | 0,045 g |
| Natriumbicarbonat | | 2,500 g |
| gereinigtes Wasser | ad | 100 ml |

zu der man außerdem etwa 1 g Natriumhydroxid zugibt, um den pH-Wert auf einen Wert von etwa 8,2 zu bringen.

23